# EUROPEAN PATENT SPECIFICATION

(11) **EP 3 288 967 B1**
(45) Date of publication and mention of the grant of the patent: **06.11.2019**
(21) Application number: 16729054.3
(22) Date of filing: 28.04.2016
(51) Int. Cl.: C07K 14/685

(54) **PHARMACEUTICAL COMPOUND**
PHARMAZEUTISCHE ZUSAMMENSETZUNG
COMPOSÉ PHARMACEUTIQUE

(30) Priority: 28.04.2015 EP 15165537
(43) Date of publication of application: 07.03.2018
(73) Proprietor: VALLAURIX PTE. LTD., 089316 Singapore (SG)
(72) Inventor: WOLGEN, Philippe, Singapore 557960 (SG); CALLENS, Roland, 8700 Tielt (BE)
(74) Representative: Farago-Schauer, Peter Andreas
(86) International application number: PCT/IB2016/052416
(87) International publication number: WO 2016/174610

(56) References cited:
- EP-A1- 2 487 185
- HOLDER JERRY RYAN ET AL: "STRUCTURE-ACTIVITY RELATIONSHIPS OF THE MELANOCORTIN TETRAPEPTIDE AC-HIS-DPHE-ARG-TRP-NH2 AT THE MOUSE MELANOCORTIN RECEPTORS PART 3: MODIFICATIONS AT THE ARG POSITION", PEPTIDES, ELSEVIER, AMSTERDAM, NL, vol. 24, no. 1, 1 January 2003 (2003-01-01), pages 73-82, XP009077304, ISSN: 0196-9781, DOI: 10.1016/S0196-9781(02)00278-4
- KLEMES D G ET AL: "Potent and prolonged melanotropic activities of the alpha-MSH fragment analog, Ac-[Nle<4>, D-Phe<7>]-alpha-MSH4-9-NH2", BIOCHEMICAL AND BIOPHYSICAL RESEARCH COMMUNICATIONS, ACADEMIC PRESS INC. ORLANDO, FL, US, vol. 137, no. 2, 13 June 1986 (1986-06-13) , pages 722-728, XP024836897, ISSN: 0006-291X, DOI: 10.1016/0006-291X(86)91138-1 [retrieved on 1986-06-13]

## Description

### Technical field

The present invention relates to a compound, a compound for use, use of a compound for manufacturing, a method of preparing a compound, a method of treating a mammal by therapy with a compound, and a method of preparing an alpha-MSH analogue.

### Background to the invention

Melanocortins include a family of peptide hormones that induce pigmentation by interaction with the melanocortin-1-Receptor (MC1R) in the epidermis. Alpha-melanocyte stimulating hormone (alpha-MSH) is a primary pigmentary hormone that is released from the pars intermedia of the pituitary gland in some non-human animals, and from UV exposed keratinocytes in human skin. This 13 amino acid peptide is represented by the formula structure Ac-Ser-Tyr-Ser-Met-Glu-His-Phe-Arg-Trp-Gly-Lys-Pro-Val-NH₂. Alpha-MSH binds to MC1R and induces cyclic AMP-mediated signal transduction leading to the synthesis of melanin polymers from DOPA precursors. Various alpha-MSH analogues have been described in WO2008025094 and WO2012107592. Holder et al (Peptides, 24:73-82, 2003) describe tetrapeptides derived from alpha-MSH.

Two types of melanin can be expressed in humans, melanin and phaeomelanin. The brownish-black pigment melanin is believed to have photoprotective properties as it is resistant to photodegradation and has the ability to quench reactive oxygen radicals. Phaeomelanin is a reddish, sulfur-containing pigment and is often expressed in light-skinned human subjects that report a poor tanning response to sunlight and are generally thought to be at a greater risk of developing both melanoma and non-melanoma skin cancers. Binding of alpha-MSH to MC1R further stimulates eumelanogenesis through activation of adenylate cyclas.

While advances have been made in treating skin and other diseases, there remains a need for more and/or improved options in the art for compounds and medical treatments.

### Summary of the invention

We have surprisingly found that modifications to alpha-MSH analogue provides for certain benefits.

According to one aspect of the invention, we have surprisingly found that the compound Ac-Nle-Glu-His-D-Phe-X-Trp-NH₂, wherein X is homoArg or norArg, or a pharmaceutically acceptable salt thereof has one or more benefits, including reduced production costs and/or efficient preparation in particular the high yield and/or the high purity level, improved activity, increased efficacy, and/or increased potency, and/or less susceptibility to degradation. The compound is a hexapeptide and an alpha-MSH analogue and provides additional benefits, particularly on a per weight basis.

In an aspect, the invention relates to a compound with formula structure Ac - Nle - Glu - His- D-Phe - homoArg - Trp - NH₂ or a pharmaceutically acceptable salt thereof. In another aspect, the invention relates to a compound with formula structure Ac - Nle - Glu - His- D-Phe - norArg - Trp - NH₂ or a pharmaceutically acceptable salt thereof. In a further embodiment, the compound of the invention is for use as a medicine. Preferably, the compound of the invention is for use in therapeutic treatment of a skin disorder. Preferably, the compound of the invention is for use in treating pigmentation disorders, photodermatoses, prevention of skin cancer, and/or DNA repair in skin cells. Preferably, the compound of the invention is applied topically to the skin.

In an aspect, the invention relates to the use of a compound according to the invention for the manufacture of a medicine.

In another aspect, the invention relates to a method of preparing compound Ac - Nle - Glu - His - D-Phe - X - Trp - NH₂, wherein X is selected from homoArg or norArg, or a pharmaceutically acceptable salt thereof, by
- providing tripeptide D-Phe - X - Trp (4-6);
- coupling tripeptide (4-6) D-Phe - X - Trp with histidine (3); and
- coupling dipeptide Nle-Glu (1-2) with tetrapeptide His - D-Phe - X - Trp (3-6).

In another aspect, the invention relates to a method wherein tripeptide D-Phe - homoArg - Trp (4-6) is prepared from tripeptide D-Phe - Lys - Trp by converting the free amino function of the Lysine side chain with guanylating reagent benzotriazole-1-carboxamidinium tosylate (BCAT).

In another aspect, the invention relates to a method of treating a mammal by therapy by administering a compound with formula structure Ac - Nle - Glu - His- D-Phe - X - Trp - NH₂, wherein X is selected from homoArg or norArg, or a pharmaceutically acceptable salt thereof.

In another aspect, the invention relates to a method of preparing an alpha-MSH analogue comprising a homoArg group by first introducing a Lysine group during the preparation of the analogue and subsequently converting the free amino function of the Lysine side chain with guanylating reagent benzotriazole-1-carboxamidinium tosylate (BCAT) to generate a homoArg group.

We have surprisingly found that the compound of the present invention provides beneficial results in in-vitro and/or in-vivo pharmaceutical tests relating to MC1R binding affinity, potency, and/or efficacy in particular for MC1R associated diseases. Further, we have found that compound of the invention can be safely and efficiently synthesized, particularly at high yield.

### Detailed description of the invention

For the purpose of the invention, the term "alpha-MSH analogue" referred to herein is defined as a derivative of alpha- MSH which exhibits agonist activity for the melanocortin-1 receptor (MC1R), the receptor to which alpha-MSH binds to initiate the production of melanin within a melanocyte.

The following abbreviations have been used in this specification Arg - arginine, D-Phe - D isomer of Phenylalanine; Glu - Glutamic acid; Gly - Glycine; His - Histidine; HomoArg - homoarginine (one additional -CH₂- unit in the alkyl chain than Arg); norArg - norarginine (one fewer-CH2 unit in the alkyl chain than Arg); Lys - Lysine; Met - Methionine; Nle - Norleucine; Phe- Phenylalanine; Ser - Serine; Trp - Tryptophan; and Tyr - Tyrosine. The prefix "D" before the amino acid designates the D-isomer configuration. Unless specifically designated otherwise, all amino acids are in the L-isomer configuration.

All peptide and peptide derivatives are written with the acylated amino terminal end at the left and the amidated carboxyl terminal at the right. As will be understood, the acylated amino terminal end may be replaced by another group according to the invention but the orientation of the peptides and peptide derivatives remains the same. Following common convention, the first amino acid on the left is located at position 1, for instance, Nle (1) indicating that Nle is positioned at the N terminal end (on the left).

In this specification, homoArg and norArg may be referred to as amino acids even though they are strictly amino acid derivatives. In the same way, compounds comprising quaternary ammonium groups, homoArg, norArg and/or other amino acid derivatives may be referred to as peptides even though they are strictly peptide derivatives. Accordingly, the skilled person will understand that reference in this document to peptide molecules (including hexapeptides and alpha-MSH analogues) includes reference to derivatives thereof.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The present invention relates to a hexapeptide alpha-MSH analogue compound with formula structure Ac-Nle-Glu-His-D-Phe-X-Trp-NH₂, wherein X is selected from homoArg or norArg, or a pharmaceutically acceptable salt thereof. According to one aspect of the invention, homoArg or norArg replaces Arg in the backbone of the alpha-MSH analogue for added benefits, including increased efficacy and provides for efficient preparation.

The compound of the invention may also be present as a pharmaceutically acceptable salt. Depending on the environment, certain amino acids may act as a base and attract a proton, resulting in a charge in the peptide, as is well known in the art. Accordingly, the compound of the invention may be in the form of a pharmaceutically acceptable salt. The compound may be a cation and be combined with a negatively charged counter-ion. Examples of pharmaceutically acceptable cation X⁺ that may be associated with the compound of the invention are ions of H⁺ (hydrogen ion), sodium, potassium, and calcium, preferably H⁺. Preferably, the counter-ion is a negatively charged pharmaceutically acceptable anion Y⁻. It will be understood that Y⁻ can also have a multiple negative charge in which case one anion may be combined with multiple positively charged compounds. Examples of pharmaceutically acceptable anion Y⁻ are derived from an organic or inorganic acid such as HCl, HBr, HI, H₂ SO₄, H₃ PO₄, acetic acid, propionic acid, glycolic acid, maleic acid, malonic acid, methanesulphonic acid, fumaric acid, succinic acid, tartaric acid, citric acid, benzoic acid, and ascorbic acid. Optionally, these compounds are halogenated, such as for instance tri-fluoroacetate. Preferably, Y⁻ is acetate, chloride or sulfate and more preferably acetate. A preferred salt is the acetic acid salt. According to the invention, the compound can be present as a salt; conversation to a salt is a standard step in the art and is optional in the present invention while it can be carried out during or after preparation of the compound.

The compound of the present invention may be used for medical indications, as medicine. It will be understood that medical indications of the invention are of a therapeutic nature. For the purpose of the invention, prevention of a disease is considered to be covered by the term treatment.

The compound of the invention may be beneficially used for treatment and/or prevention of various medical indications, preferably medical indications of an exclusive therapeutic nature. Preferably, reference to the use of the compound of the invention includes not only pharmaceutically acceptable salts, but preferably also the use of prodrugs, stereoisomers, tautomers, hydrates, hydrides and/or solvates of the compounds of the invention.

The compound of the invention can be used in the manufacture of medicines for treatment of the indications and administrations indicated in this specification.

Preferably, compounds of the invention are used for treatment of diseases wherein the compounds - through association- beneficially increase MC1R expression, as a drug target for the diseases. Examples of such diseases are pigmentation disorders, photodermatoses, skin cancer, and/or DNA repair in skin cells (after/due to UV exposure).

In one aspect, the compound of the invention is used for treatment of pigmentation (or skin pigmentation) disorders. Such disorder can either be hyperpigmentation but in this case particularly hypopigmentation disorders are important. We have found that the compound of the invention can induce melanogenesis and are useful for inducing therapeutic melanogenesis.

In an aspect, the invention relates to inducing melanogenesis in the skin as a treatment for pigmentation disorders with a compound according to the invention. The term '"melanogenesis" as used herein is defined as the ability of a subject to produce melanin by melanin-producing cells called melanocytes, for therapeutic purposes. Examples of producing therapeutic melanogenesis are protecting the skin from UV irradiation damage, for instance preventing the skin from developing wrinkles, sun burns and/or cancer.

An important example of a hypopigmentation disorder is vitiligo. Vitiligo is a chronic skin condition that is characterized by loss of pigment, including melanin, resulting in irregular pale, de-pigmented skin that has a different color and aspect than and contrast with the surrounding non-affected, pigmented, darker colored skin tissue. In an aspect, the present invention is directed to treatment of vitiligo, in particular in combination with UV light treatment.
The compound of the invention is preferred for use in the treatment of vitiligo, particularly for repigmentation of vitiliginous lesions and therefore reducing the contrast between the vitiliginous and the surrounding skin tissue.

Photodermatoses are skin diseases that are associated with photosensitivity of the skin to UV irradiation and may be classified into 5 general categories: idiopathic photodermatoses (including polymorphic light eruption (PLE), actinic prurigo, hyroa vacciniforme, chronic actinic dermatitis, and solar urticarial-SU); photodermatoses that are secondary to exogenous agents (including phototoxic and photoallergic reactions); photodermatoses secondary to endogenous agents (mainly the porphyrias including Erythropoietic PhotoPorphyria-EPP); photoexacerbated dermatoses (including autoimmune disease, infectious conditions, and nutritional deficiencies); and genodermatoses.

In an aspect, the present invention is directed to treatment of photodermatoses. The compound of the present invention is preferred for use in treatment of photodermatoses, particularly for EPP, PLE, and SU, most particularly for EPP.

Skin cancer includes melanoma and non-melanoma cancer. Generally, higher skin melanin levels are considered a measure for prevention of skin cancer. In an aspect, the present invention is directed using the compound of the invention for prevention of cancer. The compound of the invention is preferred for use in the prevention of cancer, particularly skin cancer including melanoma and particularly non-melanoma. While the general public will benefit from skin cancer prevention through the invention, certain patient groups will in particular benefit from the use of the compound of the invention, including immunocompromised patients (particularly HIV-AIDS patients, allogeneic transplant patients, i.e. the recipient receives the transplant from another subject, and/or patients on immunosuppressant medication), human subjects having one or more MC1R variant alleles associated with loss of or diminished receptor function (preferably selected from Val60LEU (V60L), Asp84Glu (D84E), Val92Met (V92M), Arg142His (R142H), Arg151Cys (R151C), Arg160Trp (R160W) and Asp294His (D294H)).

It is understood that UV irradiation can cause damage to DNA, particularly the DNA of dermal (skin) cells. In an aspect, the present invention is direct to DNA repair. Accordingly, the present invention is directed to the compound of the invention for use in DNA repair, preferably in the skin, particularly subsequent to UV irradiation of the skin.

Preferably, the compound of the invention is used on subject wherein the subject preferably being a mammal, preferably rodents and/or humans, more preferably a human subject.

In one aspect of the invention, the compound of the invention is combined with UV light for treatment of the subject.

The compound of the invention can be administered to a subject using a variety of administration or delivery techniques known in the art. The mode of administration will depend upon the subject to be treated and compound selected. In various aspects, the compound can be administered orally (or enterally), parenterally or topically (preferably to the skin).

The term "oral" is used herein to encompass administration of the compound via the digestive tract.

The term "parenteral" is used herein to encompass any route of administration, other than oral administration, by which the compound is introduced into the systemic circulation. Generally, parenteral administration can be achieved by intravenous, intramuscular, subcutaneous, intraperitoneal, intradermal, ocular, inhalable, nasal, rectal, vaginal, transdermal, buccal, sublingual, or mucosal administration.

The term "mucosal" as used herein encompasses the administration of the compound by methods that employ the mucosa (mucous membranes) of the human body such as, but not limited to, buccal, intranasal, gingival, vaginal, sublingual, pulmonary, or rectal tissue.

The term "transdermal" as used herein encompasses the administration of the compound that are applied to the skin and subsequently pass through the skin into the systemic circulation such as, but not limited to, transdermal formulations, buccal patches, skin patches, or transdermal patches.

The term "topical" as used herein encompasses administration to the skin and may include applying preparations such as creams, gels, or solutions to the skin, eye, or mucosal areas for local effect. Compounds of the invention may be incorporated into a topical composition for administered on the skin. In one aspect, the topical compositions has local efficacy in the skin at the location of application and is thus administered locally. In another aspect, the topical composition has systemic efficacy which requires the compound migrate transdermally (through the skin) into the blood stream resulting in systemic exposure to the compound and is thus administered transdermally.

Other preferred administration routes that may achieve systemic exposure to the compounds are subcutaneous ("under the skin") and intramuscular ("in the muscle").

In one aspect, the compound of the invention is topically administered to the skin. Accordingly, the invention relates to administering the compound of the invention to the skin of a subject. In another aspect, the compound of the invention is parentally administered to the skin. Accordingly, the invention relates to administering the compound of the invention through the skin of a subject.

Preferably, the compound of the invention is formulated in a composition. The composition is preferably a pharmaceutical composition. The composition preferably comprises at least one pharmaceutically-acceptable ingredient in addition to the compound of the invention. Examples of such pharmaceutically-acceptable ingredients are carriers, polymers, thickeners, diluents, fillers, buffers, preservatives, and surface active agents.

In an aspect, the composition is a controlled release formulation, resulting in longer and/or more controlled exposure of the body to the compound. The composition may be an implant. In one preferred embodiment, the compound is administered in a prolonged release implant formulation such as described in WO2006/012667.

The compound of the invention is preferably prepared as indicated below, though the skilled person will appreciate reviewing the specification that alteration of the present methods could be employed that are also covered by the presently claimed invention. According to a preferred method, the compound of the invention is prepared by liquid phase or solid phase peptide synthesis, preferably followed by chromatographic purification and preferably by lyophilisation.

In one aspect, the present invention relates to preparation of Ac - Nle - Glu - His - D-Phe - homoArg - Trp - NH₂ by:
Step 1: providing tripeptide D-Phe - X - Trp (4-6), wherein X is selected from homoArg or norArg;
Step 2: coupling tripeptide (4-6) D-Phe - X - Trp with histidine (3); and
Step 3: coupling dipeptide Nle-Glu (1-2) with tetrapeptide His - D-Phe - X - Trp (3-6).

Preferably, the compound is purified (step 4); preferably, the compound is concentrated (step 5); and preferably, the compound is lyophilizated (step 6).

Specifically, synthesis steps of the compounds of the invention comprising Ac-Nle - Glu - His - D-Phe - X - Trp-NH₂, wherein X is selected from homoArg or norArg, include the following preferred steps:
Step 1: Deprotection of tripeptide (4-6) by hydrogenolysis with a Pd/C catalyst in ethanol;
Step 2a: Deprotected tripeptide (4-6) coupling to (Fmoc) and (Trt) protected histidine (3) with HBTU/DIPEA in a dichloromethane dimethylformamide mixture;
Step 2b: Detritylation of the protected (3-6) peptide in a HOAc/H20 mixture;
Step 2c: Cleavage of the Fmoc protective group of the (3-6) peptide in a mixture of H2O/methanol and dioxane with NaOH;
Step 3: Coupling of the (1-2) dipeptide Nle-Glu(Ot.Bu) to the (3-6) peptide with DCC/HOOBt in dimethylformamide.
Step 3a: Removal of the Ot.Bu protective group from the side chain of residue 2 (Glu) by treatment with 8NHCl and phenol;
Step 4. Purification of the peptides by preparative RP-HPLC using a C-18 column and a purified water/acetonitrile/TFA eluent;
Step 5. Concentration step using the same chromatographic column with an eluent composed of the same components but with higher acetonitrile content. Organic solvents are removed by evaporation;
Step 6: Lyophilization of the aqueous solution obtained after evaporation of the organic solvents.

Each of these more specific preferred synthesis steps can independently and separately be introduced to the above general preparation method, arriving at a preferred process. Thus, each preferred step separately represents preferred conditions for the preparation of the compound of the invention.

In a preferred aspect, as will be further explained below, introduction of the homoArg group preferably occurs by first incorporating Lys and converting Lys into homoArg. Optionally, conversion of Lys to homoArg takes place in a later step of the preparation and the Lys group is temporarily protected, for instance with a trifluoro acetyl group.

The abbreviations used herein will be readily understood by the skilled person, the following list only being provided for convenience:
Ac: acetyl or CH₃-CO-
BCAT: benzotriazole-1-carboxamidinium tosylate
DCC: dicyclohexylcarbidiimide
DIPEA: diisopropylethylamine
Fmoc: fluorenylmethoxycarbonyl
HBTU: benzotriazolyl tetramethyluronium hexafluorophosphate
HOOBT: 3-hydroxy-3,4dihydro-4oxo-benzotriazine
OtBu- group: O-tert-butyl group
Trt- group: trityl group

The compound of the invention comprises a homoArg or norArg unit which may be introduced as a homoArg or norArg unit in the tripeptide of above mentioned processing step 1. However, we surprisingly found that the homoArg amino acid derivative can be beneficially introduced in alpha MSH analogues using efficient processing conditions and resulting in high yields for reduced expenses. It is noted that this aspect relates to alpha-MSH analogues generally, i.e. MC1R agonists according to the definition as provided above, comprising a homoArg group. This includes the compound of the present invention but in principle also those described in WO2008025094 of which the analogue formula structures comprising a homoArg group are incorporated herein by reference.

Accordingly, the present invention generally relates to a process of preparing an alpha-MSH analogue comprising a homoArg group by using a Lysine group and converting the Lysine group to the homoArg group by reacting the free amino function of the Lysine side chain with guanylating reagent benzotriazole-1-carboxamidinium tosylate (BCAT).

In a preferred aspect, first the tripeptide D-Phe - Lys - Trp Lysine is prepared and, subsequently, the Lysine group is converted to homoArg by reacting the free amino function of the Lysine side chain with guanylating reagent benzotriazole-1-carboxamidinium tosylate (BCAT) to generate the tripeptide D-Phe - homoArg - Trp.

Accordingly in a preferred aspect, the present invention relates to a process of preparing Ac-Nle - Glu - His - D-Phe - homoArg - Trp-NH₂ as defined above by preparing tripeptide D-Phe - homoArg - Trp of above mentioned step 1 from D-Phe - Lys - Trp by converting the free amino function of the Lysine side chain with guanylating reagent benzotriazole-1-carboxamidinium tosylate (BCAT).

Preferably, the Lysine group is introduced and converted to homoArg before above-mentioned step 1. Optionally, the Lysine group may be introduced before above mentioned step 1 but converted to homoArg in a later step in the preparation of the compound of the invention. In that case, the free amino function of the Lysine group is preferably temporarily protected. Protection can for instance be carried out with a trifluoro acetyl group. In the later step and after de-protecting, Lys is converted to homoArg with guanylating reagent benzotriazole-1-carboxamidinium tosylate (BCAT).

### Examples

The following examples are illustrative to the present invention and are presented without wishing to limit the scope of the present invention to the specific examples.

### Example 1

Specifically, the compound with formula structure Ac - Nle - Glu - His- D-Phe - homoArg - Trp - NH₂ of the present invention was generally prepared using the above mentioned processing steps 1-6. The homoarginine unit was introduced in the tripeptide before step 1 as follows: first a protected derivative of lysine was incorporate at the level of the tripeptide 4-6 before step 1, the lysine tripeptide was partially deprotected and the free amino function of the Lysine side chain was converted to a homoarginine with guanylating reagent benzotriazole-1-carboxamidinium tosylate (BCAT).

Identify and purity of the compound was confirmed by MS (not including the trifluoroacetate anion) and HPLC and the following results were obtained:

| | MW by Mass spec | HPLC purity |
|---|---|---|
| Compound | 941 | 100% |

Proof of identity was further provided with 500MHz proton spectra.
The norArg compound of the invention can be prepared as indicated above, for instance by using the norArg in the tripeptide (4-6) starting unit.

### Example 2: effects on cAMP

The compound with formula structure Ac - Nle - Glu - His- D-Phe - homoArg - Trp - NH₂ of the invention was tested using human melanocyte culture coded 1753 that expressed functional MC1R. The melanocytes were plated at a density of 0.3^{∗}10⁶ cells/ well. After 48 hours, the melanocytes were treated with different compound concentrations (10⁻¹² to 10⁻⁷ M) for 1 hour. A control without compound was included in the test. Reference compound NDP-MSH was also included as comparison at the same concentrations. The reaction was stopped by addition of 50µl 1 N HCl and the supernatant in each well was used to measure cAMP using a radioimmunoassay as described by Suzuki 1996 (Suzuki I, Cone RD, Im S, Nordlund JJ, Abdel-Malek Z: "Binding of melanotropic hormones to the MC1 receptor on human melanocytes stimulates proliferation and melanogenesis". Endocrinology 137: 1627-1633, 1996). Duplicate samples from each well were assayed with triplicate wells included in each group. The mean of 6 cAMP measurements per group was expressed as % of the control group. Statistical analysis was carried out using ANOVA followed by Newman Kuels test. In some cases, unpaired t-test was used.

The results were that the compound of the invention outperformed reference compound NDP-MSH by achieving highest efficacy (216% at 10⁻⁷ M vs 202% at 10⁻⁷ M) and the results were statistically different compared to the control at (p <0.05) at concentrations from 10⁻¹⁰ M to 10⁻⁷M.

It is concluded that the compound showed excellent efficacy results on the test measuring cAMP, the second messenger of the MC1R response.

### Example 3: effects on tyrosinase activity

The compound with formula structure Ac - Nle - Glu - His- D-Phe - homoArg - Trp - NH₂ of the invention was tested using human melanocyte culture coded 1750 that expressed functional MC1R. The melanocytes were plated at a density of 0.3^{∗}10⁶ cells onto 60mm dishes (triplicate dishes/group). After 48 hours, the melanocytes were treated every other day for a total of six days with different doses (10⁻¹² M to 10⁻⁷ M) of the compound. A control without compound was included in the test. Reference compound NDP-MSH, afamelanotide, was also included as comparison at the same concentrations. On treatment day 5, ³H-labeled tyrosine, the substrate for tyrosinase, was added and 24 hours later, the supernatant was saved to be assayed for tyrosinase activity as described by Suzuki et al (see example 2). Duplicate samples from each were assayed, with triplicate dishes included in each group. Cell number in each dish was counted, and tyrosinase activity was expressed as dpm/10⁶ cells and as % of the control. Statistical analysis was carried out using ANOVA followed by Newman Kuels test.

It will be understood that this tyrosinase activation test relates to a late event after MC1R activation, compared to the earlier secondary messenger effect of the above cAMP test. As pointed out above, activity of tyrosinase requires days of treatment.

We found that the compound outperformed reference compound NDP-MSH, afamelanotide, by achieving highest efficacy (227% at 10⁻⁸ M vs 156% at 10⁻⁹ M; with the compound actually also having a higher efficacy of 198% at 10⁻⁹ M) and the results were statistically different compared to the control (at p <0.05) at concentrations from 10⁻¹⁰ M to 10⁻⁷M.

It is concluded that the compound of the invention showed excellent efficacy on the test measuring tyrosinase, representing a late event following agonist activity on the MC1R.

It will be appreciated by persons skilled in the art that numerous variations and/or modifications may be made to the invention as shown in the specific embodiments. The present embodiments are, therefore, to be considered in all respects as illustrative and not restrictive.

## Claims

1. Compound with formula structure Ac - Nle - Glu - His- D-Phe -X - Trp - NH₂, wherein X is homoArg or norArg, or a pharmaceutically acceptable salt thereof.

2. Compound according to claim 1 wherein X is homoArg.

3. Compound according to claim 1 wherein X is norArg.

4. Compound according to claims 1-3 for use as a medicine.

5. Compound according to claims 1-4 for use in therapeutic treatment of a skin disorder.

6. Compound according to claims 1-5, for use in treating pigmentation disorders, photodermatoses, prevention of skin cancer, and/or DNA repair in skin cells.

7. Compound for use according to claims 4-6, wherein the compound is applied topically to the skin.

8. Method of preparing compound Ac - Nle - Glu - His - D-Phe - X - Trp - NH₂, wherein X is homoArg or norArg, or a pharmaceutically acceptable salt thereof, by
- providing tripeptide D-Phe - X - Trp (4-6);
- coupling tripeptide (4-6) D-Phe - X - Trp with histidine (3); and
- coupling dipeptide Nle-Glu (1-2) with tetrapeptide His - D-Phe - X - Trp (3-6).

9. Method according to claim 8, wherein tripeptide D-Phe - homoArg - Trp (4-6) is prepared from tripeptide D-Phe - Lys - Trp by converting the free amino function of the Lysine side chain with guanylating reagent benzotriazole-1-carboxamidinium tosylate (BCAT).

10. Method of preparing an alpha-MSH analogue according to claim 1 comprising tripeptide D-Phe - homoArg - Trp with a homoArg group by first introducing a Lysine group as a tripeptide D-Phe - Lys - Trp during the preparation of the analogue, then converting the free amino function of the Lysine side chain with guanylating reagent benzotriazole-1-carboxamidinium tosylate (BCAT) to generate a homoArg group and subsequently using the tripeptide D-Phe - homoArg - Trp to prepare the alpha-MSH analogue.

## Patentansprüche

1. Eine Verbindung mit der Formelstruktur Ac - Nle - Glu - His - D-Phe - X - Trp - NH₂, worin X homoArg oder norArg ist, oder ein pharmazeutisch verträgliches Salz davon.

2. Die Verbindung gemäß Anspruch 1, worin X homoArg ist.

3. Die Verbindung gemäß Anspruch 1, worin X norArg ist.

4. Die Verbindung gemäß Ansprüchen 1-3 für die Verwendung als ein Medikament.

5. Die Verbindung gemäß Ansprüchen 1-4 für die Verwendung in der therapeutischen Behandlung einer Hautkrankheit.

6. Die Verbindung gemäß Ansprüchen 1-5, für die Verwendung in der Behandlung von Pigmentierungsstörungen, Photodermatosen, der Verhinderung von Hautkrebs, und/oder der DNA-Reparatur in Hautzellen.

7. Die Verbindung für die Verwendung gemäß Ansprüchen 4-6,
worin die Verbindung topisch auf die Haut aufgetragen wird.

8. Ein Verfahren zur Herstellung der Verbindung Ac - Nle - Glu - His - D-Phe - X - Trp - NH₂, worin X homoArg oder norArg ist, oder ein pharmazeutisch verträgliches Salz davon, durch
- Bereitstellen des Tripeptids D-Phe - X - Trp (4-6);
- Koppeln des Tripeptids (4-6) D-Phe - X - Trp mit Histidin (3); und
- Koppeln des Dipeptids Nle-Glu (1-2) mit dem Tetrapeptid His - D-Phe - X - Trp (3-6).

9. Das Verfahren gemäß Anspruch 8, worin das Tripeptid D-Phe - homoArg - Trp (4-6) aus dem Tripeptid D-Phe - Lys - Trp durch Umwandeln der freien Aminofunktion der Lysin-Seitenkette mit dem Guanylierungsreagenz Benzotriazol-1-carboxamidiniumtosylat (BCAT) hergestellt wird.

10. Ein Verfahren zur Herstellung eines alpha-MSH Analogs gemäß Anspruch 1, das ein Tripeptid D-Phe - homoArg - Trp mit einer homoArg-Gruppe umfasst, indem während der Herstellung des Analogs zuerst eine Lysingruppe als Tripeptid D-Phe - Lys - Trp eingeführt wird und danach die freie Aminofunktion der Lysinseitenkette mit dem Guanylierungsreagenz Benzotriazol-1-carboxamidiniumtosylat (BCAT) umgewandelt wird, um eine homoArg-Gruppe zu erzeugen, und anschließend das Tripeptid D-Phe - homoArg - Trp verwendet wird, um das alpha-MSH-Analog herzustellen.

## Revendications

1. Composé ayant la structure de formule Ac - Nle - Glu - His - D-Phe - X - Trp - NH₂, dans laquelle X représente homoArg or norArg ou un sel pharmaceutiquement acceptable de ceux-ci.

2. Composé selon la revendication 1, dans lequel X représente homoArg.

3. Composé selon la revendication 1, dans lequel X représente norArg.

4. Composé selon l'une quelconque des revendications 1 à 3 destiné à être utilisé en tant que médicament.

5. Composé selon l'une quelconque des revendications 1 à 4 destiné à être utilisé dans un traitement thérapeutique d'un trouble de la peau.

6. Composé selon l'une quelconque des revendications 1 à 5 destiné à être utilisé dans le traitement de troubles liés à la pigmentation, de photodermatoses, de la prévention du cancer de la peau et/ou de la réparation de l'ADN dans les cellules de la peau.

7. Composé destiné à être utilisé selon l'une quelconque des revendications 4 à 6, dans lequel le composé est appliqué de manière topique sur la peau.

8. Procédé de préparation d'un composé ayant la structure Ac - Nle - Glu - His - D-Phe - X - Trp - NH₂, dans laquelle X représente homoArg or norArg ou un sel pharmaceutiquement acceptable de ceux-ci, consistant à
- fournir le tripeptide D-Phe - X - Trp (4-6);
- coupler le tripeptide (4-6) D-Phe - X - Trp avec de l'histidine (3); et
- coupler le dipeptide Nle - Glu (1-2) avec le tétrapeptide His - D-Phe - X -Trp (3-6).

9. Procédé selon la revendication 8, dans lequel le tripeptide D-Phe - homoArg - Trp (4-6) est préparé à partir du tripeptide D-Phe - Lys - Trp par conversion de la fonction amino libre de la chaîne latérale de la lysine avec le réactif de guanylation, le tosylate de benzotriazole-1-carboxamidinium (BCAT).

10. Procédé de préparation d'un analogue alpha-MSH selon la revendication 1 comprenant un tripeptide D-Phe - homoArg - Trp avec un groupe homoArg en introduisant d'abord un groupe lysine comme un tripeptide D-Phe - Lys - Trp pendant la préparation de l'analogue, ensuite en convertissant la fonction amino libre de la chaîne latérale de la lysine avec le réactif de guanylation, le tosylate de benzotriazole-1-carboxamidinium (BCAT) afin de générer un groupe homoArg et par la suite en utilisant le tripeptide D-Phe - homoArg - Trp pour préparer l'analogue alpha-MSH.
